# EUROPEAN PATENT SPECIFICATION

(11) **EP 4 271 356 B1**
(45) Date of publication and mention of the grant of the patent: **03.06.2026**
(21) Application number: 21834952.0
(22) Date of filing: 30.11.2021
(51) Int. Cl.: A61K 8/365, A61K 8/64, A61Q 5/12, A61K 8/73, A61K 8/84, A61K 8/97

(54) **COMPOSITION FOR KERATIN FIBERS**
ZUSAMMENSETZUNG FÜR KERATINFASERN
COMPOSITION POUR LES FIBRES KÉRATINIQUES

(30) Priority: 01.12.2020 JP 2020199437; 11.01.2021 FR 2100202
(43) Date of publication of application: 08.11.2023
(73) Proprietor: L'OREAL, 75008 Paris (FR)
(72) Inventor: CHAUMONTET, Manon, 93400 Saint-Ouen (FR); KAESER, Adrien, Kawasaki-shi, Kanagawa 2130012 (JP); XING, Tong, Kawasaki-shi, Kanagawa 2130012 (JP)
(74) Representative: Lavoix
(86) International application number: PCT/JP2021/044601
(87) International publication number: WO 2022/118980

(56) References cited:
- DE-A1- 10 048 922
- DE-A1- 19 905 768
- DATABASE GNPD [online] MINTEL; 27 July 2020 (2020-07-27), ANONYMOUS: "Shampoo", XP055867364, retrieved from https://www.gnpd.com/sinatra/recordpage/7982895/ Database accession no. 7982895
- DATABASE GNPD [online] MINTEL; 25 March 2020 (2020-03-25), ANONYMOUS: "Shampoo", XP055867367, retrieved from https://www.gnpd.com/sinatra/recordpage/7462667/ Database accession no. 7462667
- DATABASE GNPD [online] MINTEL; 19 November 2020 (2020-11-19), ANONYMOUS: "Reconstructive Shampoo", XP055867359, retrieved from https://www.gnpd.com/sinatra/recordpage/8280311/ Database accession no. 8280311
- DATABASE GNPD [online] MINTEL; 6 March 2020 (2020-03-06), ANONYMOUS: "Gel Shampoo", XP055867371, retrieved from https://www.gnpd.com/sinatra/recordpage/7320805/ Database accession no. 7320805
- DATABASE GNPD [online] MINTEL; 19 November 2020 (2020-11-19), ANONYMOUS: "Reconstructive Shampoo", XP055867359, retrieved from https://www.gnpd.com/sinatra/recordpage/8280311/ Database accession no. 8280311
- DATABASE GNPD [online] MINTEL; 27 July 2020 (2020-07-27), ANONYMOUS: "Shampoo", XP055867364, retrieved from https://www.gnpd.com/sinatra/recordpage/7982895/ Database accession no. 7982895
- DATABASE GNPD [online] MINTEL; 25 March 2020 (2020-03-25), ANONYMOUS: "Shampoo", XP055867367, retrieved from https://www.gnpd.com/sinatra/recordpage/7462667/ Database accession no. 7462667
- DATABASE GNPD [online] MINTEL; 6 March 2020 (2020-03-06), ANONYMOUS: "Gel Shampoo", XP055867371, retrieved from https://www.gnpd.com/sinatra/recordpage/7320805/ Database accession no. 7320805
- SEPPIC: "Hair care formulary, Nourishing and moisturizing hair care", COSMETICS & TOILETRIES MAGAZINE, ALLURED PUBLISHING CORP, US, vol. 120, no. 11, 1 November 2005 (2005-11-01), pages 2 - 7, XP002364662, ISSN: 0361-4387

## Description

### TECHNICAL FIELD

The present invention relates to a composition for treating, such as conditioning, keratin fibers such as hair.

### BACKGROUND ART

In the field of hair cosmetics, a hair conditioning effect is a very important property. A variety of leave-on type and rinse-off type hair care cosmetic products have been used for conditioning hair.

WO 2020/085268 discloses a composition for increasing silicone deposition on keratin fibers such as hair, comprising tartaric acid, aromatic alcohol, amino-modified silicone and organopolysiloxane selected from polydialkylsiloxane, polydiarylsiloxane, and polyalkylarylsiloxane. The deposition of silicone on keratin fibers can smoothen hair.

### DISCLOSURE OF INVENTION

There is still a need for hair care cosmetic products with good cosmetic effects based on a new approach. In particular, there is a need for hair care cosmetic products for smoothening hair even without depending on silicone.

An objective of the present invention is to provide a composition which can smoothen keratin fibers based on a new approach.

The above objective of the present invention can be achieved by a composition for treating keratin fibers, preferably hair, comprising:
(a) at least one α-hydroxy acid or salt thereof;
(b) at least one cationic polyamino acid comprising at least one quaternary ammonium group and being derived from plant proteins; and
(c) water,
wherein
the amount of the (a) α-hydroxy acid or salt thereof in the composition is 1% by weight or more, relative to the total weight of the composition,
the amount of the (b) cationic polyamino acid(s) is 0.2% by weight or more, preferably 0.3% by weight or more, more preferably 0.5% by weight or more, and even more preferably 0.8% by weight or more, relative to the total weight of the composition, and
the amount of silicone (s) in the composition is 1% by weight or less, relative to the total weight of the composition.

The (a) α-hydroxy acid may have two or more carboxylic groups.

The (a) α-hydroxy acid may be selected from the group consisting of glycolic acid, lactic acid, malic acid, citric acid, tartaric acid, mandelic acid, gluconic acid, mucic acid, and a mixture thereof, and more preferably from the group consisting of citric acid, tartaric acid, and a mixture thereof.

The citric acid or its salts may be derived from natural sources including but not limiting to Amanatsu, Balady citron, Bergamot orange, bitter orange, blood orange, buddha's hand, calamondin, Cam sành, chinotto, citrange, citron, citrumelo, clementine, Corsican citron, desert lime, Etrog, finger lime, Florentine citron, grapefruit, greek citron, haruka, hassaku, hyuganatsu, first lady, Jabara, kabosu, kaffir lime, kanpei, kawachi bankan, key lime, kinkoji unshiu, kinnow, Kiyomi, kobayashi mikan, koji orange, kuchinotsu No.37, Kumquat, lemon, lime, lumia, mandarin orange, Mangshanyegan, meyer lemon, Moroccan citron, Myrtle-leaved orange tree, navel orange, orange, orangelo, oroblanco, papeda, Persian lime, pomelo, pompia, ponkan, ponderosa lemon, Rangpur, round lime, satsuma, shangjuan, shonan gold, sudachi, sweet limetta, Taiwan tangerine, tangelo, tangerine, tangor, ugli fruid, volkamer lemon, yukou, yuzu, blueberry, strawberry, raspberry, cranberry, red currant, black currant, gooseberry, pineapple, tamarind, cherry, peach, apricot and tomato.

The tartaric acid or its salts may be derived from natural sources including but not limiting to grape, apricot, apple, banana, avocado and tamarind.

The amount of the (a) α-hydroxy acid(s) or salt thereof in the composition according to the present invention may be from 1% to 10% by weight, relative to the total weight of the composition.

The (b) cationic polyamino acid is derived from plant proteins.

The cationic moiety of the (b) cationic polyamino acid comprises at least one quaternary ammonium group.

The (b) cationic polyamino acid may be selected from the group consisting of hydroxypropyl trimonium hydrolyzed wheat protein, steardimonium hydroxypropyl hydrolyzed wheat protein, cocodimonium hydroxypropyl hydrolyzed wheat protein, hydroxypropyltrimonium hydrolyzed conchiolin protein, steardimonium hydroxypropyl hydrolyzed soy protein, hydroxypropyltrimonium hydrolyzed soy protein, cocodimonium hydroxypropyl hydrolyzed soy protein, and a mixture thereof.

The amount of the (b) cationic polyamino acid(s) in the composition according to the present invention may be 20% by weight or less, preferably 15% by weight or less, more preferably 10% by weight or less, and even more preferably 5% by weight or less, relative to the total weight of the composition.

The amount of the (c) water in the composition according to the present invention may be from 50% to 80% by weight, preferably from 55% to 75% by weight, and more preferably from 60% to 70% by weight, relative to the total weight of the composition.

The pH of the composition according to the present invention may be less than 7, preferably less than 6, and more preferably less than 5.

The composition according to the present invention may further comprise (d) at least one cationic surfactant which is different from the (b) cationic polyamino acid.

The composition according to the present invention may further comprise (e) at least one nonionic surfactant.

The composition according to the present invention may be a cosmetic composition, preferably a rinse-off type cosmetic composition, and more preferably a rinse-off type hair cosmetic composition.

The present invention also relates to a cosmetic process for caring for or conditioning keratin fibers, preferably hair, comprising the step of:
applying onto the keratin fibers the composition according to the present invention.

### BEST MODE FOR CARRYING OUT THE INVENTION

After diligent research, the inventors have discovered that it is possible to provide a composition which can smoothen keratin fibers based on a new approach.

The inventors surprisingly discovered that the use of a combination of (a) at least one α-hydroxyl acid and (b) at least one cationic polyamino acid in a composition comprising (c) water, wherein the amount of the (b) cationic polyamino acid(s) in the composition is 0.2% by weight or more, preferably 0.3% by weight or more, more preferably 0.5% by weight or more, and even more preferably 0.8% by weight or more, relative to the total weight of the composition, can smoothen keratin fibers such as hair, when the composition is applied onto the keratin fibers.

Thus, the composition for treating keratin fibers, preferably hair, according to the present invention comprises:
(a) at least one α-hydroxy acid or salt thereof;
(b) at least one cationic polyamino acid comprising at least one quaternary ammonium group and being derived from plant proteins; and
(c) water,
wherein
the amount of the (a) α-hydroxy acid or salt thereof in the composition is 1% by weight or more, relative to the total weight of the composition,
the amount of the (b) cationic polyamino acid(s) is 0.2% by weight or more, preferably 0.3% by weight or more, more preferably 0.5% by weight or more, and even more preferably 0.8% by weight or more, relative to the total weight of the composition, and
the amount of silicone (s) in the composition is 1% by weight or less, relative to the total weight of the composition.

The present invention can smoothen keratin fibers. The smoothening effect of the present invention does not depend on silicone. Therefore, the present invention is environmental friendly.

The present invention can provide good cosmetic effects, such as smoothening, easy combing and softening of keratin fibers. Even if keratin fibers are wet, the present invention can provide the keratin fibers with good cosmetic effects. Preferably, the present invention can provide good cosmetic effects in terms of all of smoothening, easy combing and softening of keratin fibers.

It is possible for the present invention to provide long-lasting good cosmetic effects such as smoothening, easy combing, and softness of keratin fibers.

The present invention can contribute to an attractive feeling to the touch, e.g., good feeling when running fingers through keratin fibers. The stickiness of keratin fibers can also be prevented or reduced.

In addition, the present invention can reinforce keratin fibers. The reinforcement of keratin fibers can increase the denaturation temperature of the keratin fibers.

The "keratin fibers" here mean fibers which include at least one keratin substance. It is preferable that at least a part of the surface of the keratin fibers be formed by keratin substances. Examples of keratin fibers include hair, eyebrows, eyelashes, and the like. It is preferable that the present invention be used for treating hair.

Hereafter, the present invention will be described in a detailed manner.

### [Composition]

The present invention relates to a composition for treating keratin fibers, preferably hair, comprising:
(a) at least one α-hydroxy acid or salt thereof;
(b) at least one cationic polyamino acid comprising at least one quaternary ammonium group and being derived from plant proteins; and
(c) water,
wherein
the amount of the (a) α-hydroxy acid or salt thereof in the composition is 1% by weight or more, relative to the total weight of the composition,
the amount of the (b) cationic polyamino acid(s) is 0.2% by weight or more, preferably 0.3% by weight or more, more preferably 0.5% by weight or more, and even more preferably 0.8% by weight or more, relative to the total weight of the composition, and
the amount of silicone (s) in the composition is 1% by weight or less, relative to the total weight of the composition.

### (Hydroxy Acid)

The composition according to the present invention comprises at least one (a) α-hydroxy acid or salt thereof. If two or more (a) α-hydroxy acids or salts thereof are used, they may be the same or different.

The (a) α-hydroxy acid may comprise at least one mono- or polycarboxylic acid comprising at least one hydroxyl functional group. The α position reflects the fact that at least one of the hydroxyl functions occupies an α position relative to at least one of the carboxyl functions of the acid, i.e., it is attached to the carbon bearing the hydroxyl function. The acid may be present in a form chosen from free acids, associated salts thereof (such as salts with organic bases and alkali metals), for example, depending on the final pH given to the composition, and optionally the corresponding lactides (i.e., the form obtained by self-esterification of the molecules).

### AHA:

The composition according to the present invention comprises at least one α-hydroxy acid (AHA) or salt thereof. If two or more α-hydroxy acids or salts thereof are used, they may be the same or different.

The term "α-hydroxy acid", or "AHA", here means a carboxylic acid which has at least one hydroxyl group on the adjacent (alpha) carbon atom.

The α-hydroxy acid may be represented by the following chemical formula:

(Rₐ)(R_{b})C(OH)COOH

where
Rₐ and R_{b} are H, F, Cl, Br, I, alkyl, aralkyl or aryl group of saturated or unsaturated, isomeric or non-isomeric, straight or branched chain or cyclic form, having 1 to 25 carbon atoms, and in addition Rₐ and R_{b} may carry OH, CHO, COOH and an alkoxyl group having 1 to 9 carbon atoms.

The hydrogen atom attached to the carbon atom may be substituted by F, Cl, Br, I, or a lower alkyl, aralkyl, aryl or alkoxyl group having 1 to 9 carbon atoms. The alpha hydroxy acids may be present as a free acid or lactone form, or in a partial salt form with an organic base or an inorganic alkali. The alpha hydroxy acids may exist as stereoisomers such as D, L, DL and meso forms.

When Rₐ and R_{b} are alkyl, they independently can be within any of the groups of C₁-C₅, C₆-C₁₀, C₁₁-C₁₅, C₁₆-C₂₀, C₂₁-C₂₅ and C₂₆-C₂₉. Compounds within the above chemical formula thus include all of the possible combinations of Rₐ and R_{b}. Included within the foregoing is a subgenus of compounds having Rₐ and R_{b} independently selected from C₁-C₁₂.

Typical alkyl, aralkyl, aryl and alkoxyl groups for Rₐ and R_{b} include methyl, ethyl, propyl, propyl, isopropyl, butyl, pentyl, octyl, lauryl, stearyl, benzyl, phenyl, methoxyl, and ethoxyl.

The alpha hydroxy acids of the first group may be subdivided into
(1) alkyl alpha hydroxy acids,
(2) aralkyl and aryl alpha hydroxy acids,
(3) polyhydroxy alpha hydroxy acids,
(4) polycarboxylic alpha hydroxy acids, and
(5) miscellaneous alpha hydroxy acids.

The following are representative alpha hydroxy acids in each subgroup.
(1) Alkyl Alpha Hydroxy Acids: 2-hydroxyethanoic acid (glycolic acid), 2-hydroxypropanoic acid (lactic acid), 2-methyl 2-hydroxypropanoic acid (methyllactic acid), 2-hydroxybutanoic acid, 2-hydroxypentanoic acid, 2-hydroxyhexanoic acid, 2-hydroxyheptanoic acid, 2-hydroxyoctanoic acid, 2-hydroxynonanoic acid, 2-hydroxydecanoic acid, 2-hydroxyundecanoic acid, 2-hydroxydodecanoic acid, 2-hydroxytetradecanoic acid, 2-hydroxyhexadecanoic acid, 2-hydroxyoctadecanoic acid, 2-hydroxyeicosanoic acid (alpha hydroxyarachidonic acid), 2-hydroxytetraeicosanoic acid (cerebronic acid), 2-hydroxytetraeicosenoic acid (alpha hydroxynervonic acid) and 2,4-dihydroxy-3,3-dimethylbutanoic acid (pantoic acid)
(2) Aralkyl and Aryl Alpha Hydroxy Acids: 2-phenyl 2-hydroxyethanoic acid (mandelic acid); 2,2-diphenyl 2-hydroxyethanoic acid (benzilic acid), 3-phenyl 2-hydroxypropanoic acid (phenyllactic acid), 2-phenyl 2-methyl 2-hydroxyethanoic acid (atrolactic acid) and 4-hydroxymandelic acid.
(3) Polyhydroxy Alpha Hydroxy Acids: 2,3-dihydroxypropanoic acid (glyceric acid); 2,3,4-trihydroxybutanoic acid (isomers; erythronic acid, threonic acid); 2,3,4,5-tetrahydroxypentanoic acid (isomers; ribonic acid, arabinoic acid, xylonic acid, lyxonic acid); 2,3,4,5,6-pentahydroxyhexanoic acid (isomers; allonic acid, altronic acid, gluconic acid, mannoic acid, gulonic acid, idonic acid, galactonic acid, talonic acid); 2,3,4,5,6,7-hexahydroxyheptanoic acid (isomers; glucoheptonic acid, galactoheptonic acid, mannoheptonic acid, etc.)
(4) Polycarboxylic Alpha Hydroxy Acids: 2-hydroxypropane-1,3-dioic acid (tartronic acid); 2-hydroxybutane-1,4-dioic acid (malic acid); 2-hydroxy-2-methylbutane-1,4-dioic acid (citramalic acid); 2,3-dihydroxybutane-1,4-dioic acid (tartaric acid); 2,3,4-trihydroxypentane-1,5-dioic acid (isomers; ribaric acid, arabaric acid, xylaric acid, lyxaric acid); 2,3,4,5-tetrahydroxyhexane-1,6-dioic acid (isomers; glucaric acid, galactaric acid, mannaric acid, allaric acid, altraric acid, gularic acid, idaric acid, talaric acid); 2-hydroxy-1,2,3-propanetricarboxylic acid (citric acid); 1-hydroxy-1,2,3-propanetricarboxylic acid (isocitric acid); 1-hydroxy-1,2,4-butanetricarboxylic acid (homoisocitric acid); 2-hydroxy-3-hexadecyl-1,2,3-propanetricarboxylic acid (n-hexadecyl citric acid; agaricic acid).
(5) Miscellaneous Alpha Hydroxy Acids: glyceruronic acid, erythruronic acid, threuronic acid; 2,3,4-trihydroxypentanuronic acids (isomers; riburonic acid, arabinuronic acid, xyluronic acid, lyxuronic acid); 2,3,4,5-tetrahydroxyhexanuronic acid (isomers; alluronic acid, altruronic acid, glucuronic acid, mannuronic acid, guluronic acid, iduronic acid, galacturonic acid, taluronic acid); 2,3,4,5,6-pentahydroxyheptanuronic acid (isomers; alloheptanuronic acid, altroheptanuronic acid, glucoheptanuronic acid, mannoheptanuronic acid, guloheptanuronic acid, idoheptanuronic acid, galactoheptanuronic acid, taloheptanuronic acid).

The α-hydroxy acid may be selected from, for example, the group consisting of glycolic acid, lactic acid, malic acid, citric acid, tartaric acid, mandelic acid, gluconic acid, mucic acid, and a mixture thereof.

Preferred α-Hydroxy Acid:
It is preferable that the (a) α-hydroxy acid have two or more carboxylic groups, and more preferably two or three carboxylic groups.

It is also preferable that the (a) α-hydroxy acid have one or more hydroxyl groups, and more preferably one or two hydroxyl groups.

It is more preferable that the (a) α-hydroxyl acid be selected from the group consisting of glycolic acid, lactic acid, malic acid, citric acid, tartaric acid, mandelic acid, gluconic acid, mucic acid, and a mixture thereof.

It is even more preferable that the (a) α-hydroxyl acid be selected from the group consisting of citric acid, tartaric acid, and a mixture thereof.

The citric acid or its salts may be derived from natural sources including but not limiting to Amanatsu, Balady citron, Bergamot orange, bitter orange, blood orange, buddha's hand, calamondin, Cam sành, chinotto, citrange, citron, citrumelo, clementine, Corsican citron, desert lime, Etrog, finger lime, Florentine citron, grapefruit, greek citron, haruka, hassaku, hyuganatsu, first lady, Jabara, kabosu, kaffir lime, kanpei, kawachi bankan, key lime, kinkoji unshiu, kinnow, Kiyomi, kobayashi mikan, koji orange, kuchinotsu No.37, Kumquat, lemon, lime, lumia, mandarin orange, Mangshanyegan, meyer lemon, Moroccan citron, Myrtle-leaved orange tree, navel orange, orange, orangelo, oroblanco, papeda, Persian lime, pomelo, pompia, ponkan, ponderosa lemon, Rangpur, round lime, satsuma, shangjuan, shonan gold, sudachi, sweet limetta, Taiwan tangerine, tangelo, tangerine, tangor, ugli fruid, volkamer lemon, yukou, yuzu, blueberry, strawberry, raspberry, cranberry, red currant, black currant, gooseberry, pineapple, tamarind, cherry, peach, apricot and tomato.

The tartaric acid or its salts may be derived from natural sources including but not limiting to grape, apricot, apple, banana, avocado and tamarind.

The (a) α-hydroxyl acid(s) or salt(s) thereof is present in the composition according to the present invention in an amount of 1% by weight or more, relative to the total weight of the composition.

The (a) α-hydroxyl acid(s) or salt(s) thereof may be present in the composition according to the present invention in an amount of 20% by weight or less, preferably 15% by weight or less, and more preferably 10% by weight or less, relative to the total weight of the composition.

The (a) α-hydroxyl acid(s) or salt(s) thereof may be present in the composition according to the present invention in an amount ranging from 1% to 10% by weight, relative to the total weight of the composition.

### (Cationic Polyamino Acid)

The composition according to the present invention comprises (b) at least one cationic polyamino acid. Two or more (b) cationic polyamino acids may be used in combination. Thus, a single type of cationic polyamino acid or a combination of different types of cationic polyamino acids may be used.

The (b) cationic polyamino acid has a positive charge density. The charge density of the (b) cationic polyamino acid may be from 0.01 meq/g to 20 meq/g, preferably from 0.05 to15 meq/g, and more preferably from 0.1 to 10 meq/g.

It may be preferable that the molecular weight of the (b) cationic polyamino acid be 1,000 or more, preferably 5,000 or more, more preferably 10,000 or more, and even more preferably 20,000 or more, and/or 1,000,000 or less, more preferably 500,000 or less, and even more preferably 100,000 or less.

Unless otherwise defined in the descriptions, "molecular weight" means a number-average molecular weight (for non-proteins) or daltons (for proteins).

The (b) cationic polyamino acid has at least one positively charged moiety. The positively charged moiety comprises at least one quaternary ammonium group, preferably at least one trialkylammonium group, and more preferably at least one trimethylammonium group. Thus, the (b) cationic polyamino acid comprises at least one quaternary ammonium group, preferably at least one trialkylammonium group, and more preferably at least one trimethylammonium group.

The positively charged moiety may also have at least one hydroxyl group, preferably one hydroxyl group. Thus, for example, the positively charged moiety may be a hydroxypropyltrimethylammonium group.

The (b) cationic polyamino acid may be cationic homopolymers or copolymers, with a plurality of amino groups and carboxyl groups. The amino group may be a primary, secondary, and tertiary amino group. The amino group may be present in a polymer backbone or a pendent group, if present, of the (b) cationic polyamino acid. The carboxyl group may be present in a pendent group, if present, of the (b) cationic polyamino acids.

As examples of the (b) cationic polyamino acids, mention may be made of hydroxypropyl trimonium hydrolyzed wheat protein, steardimonium hydroxypropyl hydrolyzed wheat protein, cocodimonium hydroxypropyl hydrolyzed wheat protein, hydroxypropyltrimonium hydrolyzed conchiolin protein, steardimonium hydroxypropyl hydrolyzed soy protein, hydroxypropyltrimonium hydrolyzed soy protein, cocodimonium hydroxypropyl hydrolyzed soy protein, and a mixture thereof.

The (b) cationic polyamino acid used in the composition according to the present invention is not a synthetic cationic polymer. Thus, the (b) cationic polyamino acid is from natural origins.

Tthe (b) cationic polyamino acid is derived from plant proteins. It is preferable that the (b) cationic polymino acid be selected from the group consisting of hydroxypropyl trimonium hydrolyzed wheat protein, steardimonium hydroxypropyl hydrolyzed wheat protein, cocodimonium hydroxypropyl hydrolyzed wheat protein, hydroxypropyltrimonium hydrolyzed conchiolin protein, steardimonium hydroxypropyl hydrolyzed soy protein, hydroxypropyltrimonium hydrolyzed soy protein, cocodimonium hydroxypropyl hydrolyzed soy protein, and a mixture thereof.

The amount of the (b) cationic polyamino acid(s) in the composition according to the present invention is 0.2% by weight or more, preferably 0.3% by weight or more, more preferably 0.5% by weight or more, and even more preferably 0.8% by weight or more, relative to the total weight of the composition.

The amount of the (b) cationic polyamino acid(s) in the composition according to the present invention may be 20% by weight or less, preferably 15% by weight or less, more preferably 10% by weight or less, and even more preferably 5% by weight or less, relative to the total weight of the composition.

The amount of the (b) cationic polyamino acid(s) in the composition according to the present invention may be from 0.2% to 20% by weight, preferably from 0.3% to 15% by weight, more preferably from 0.5% to 10% by weight, and even more preferably from 0.8% to 5% by weight, relative to the total weight of the composition.

### (Water)

The composition according to the present invention comprises (c) water.

The amount of the (c) water may be 50% by weight or more, preferably 55% by weight or more, and more preferably 60% by weight or more, relative to the total weight of the composition.

The amount of the (c) water may be 80% by weight or less, preferably 75% by weight or less, and more preferably 70% by weight or less, relative to the total weight of the composition.

The amount of the (c) water may be from 50% to 80% by weight, preferably from 55% to 75% by weight, and more preferably from 60% to 70% by weight, relative to the total weight of the composition.

### (pH)

The composition according to the present invention may have a pH of less than 7, preferably less than 6, and more preferably less than 5. The pH of the composition according to the present invention may be adjusted to be, for example, from 3 to less than 7, preferably from 3 to less than 6, and more preferably from 3 to less than 5, such as 4±0.5. The pH of the composition according to the present invention can be determined by measuring the pH of the aqueous phase of the composition.

In other words, it is preferable that the composition according to the present invention be acidic.

The pH of the composition according to the present invention may be controlled by adding at least one pH adjuster to the composition.

The pH adjuster may be selected from organic or inorganic bases and organic or inorganic acids, as well as salts thereof.

Examples of the organic base include primary, secondary and tertiary (poly) amines, such as monoethanolamine, diethanolamine, triethanolamine, isopropanolamine and 1,3-propanediamine. Examples of the inorganic base include ammonia, ammonium hydroxide, sodium hydroxide, potassium hydroxide and sodium carbonate.

Examples of the organic acid include carboxylic acid such as citric acid and lactic acid. Examples of the inorganic acid include hydrochloric acid, nitric acid, orthophosphoric acid and sulphonic acid. Examples of the salt includes sodium phosphate and trisodium phosphate.

The pH adjuster(s) may be present in the composition according to the present invention in an amount sufficient to adjust the pH of the composition to the desired value, for example, ranging from 0.01% to 10% by weight, preferably from 0.05% to 5% by weight, and more preferably from 0.1% to 1% by weight relative to the total weight of the composition.

### (Cationic Surfactant)

The composition according to the present invention may comprise (d) at least one cationic surfactant. If two or more (d) cationic surfactants are used, they may be the same or different.

The (d) cationic surfactant used in the present invention is different from the (b) cationic polyamino acid.

The cationic surfactant may be selected from the group consisting of optionally polyoxyalkylenated, primary, secondary or tertiary fatty amine salts, quaternary ammonium salts, and mixtures thereof.

Examples of quaternary ammonium salts that may be mentioned include, but are not limited to:
those of general formula (B3) below: 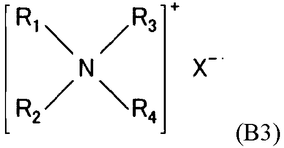 wherein
R₁, R₂, R₃, and R₄, which may be identical or different, are chosen from linear and branched aliphatic radicals including from 1 to 30 carbon atoms and optionally including heteroatoms such as oxygen, nitrogen, sulfur, and halogens. The aliphatic radicals may be chosen, for example, from alkyl, alkoxy, C₂-C₆ polyoxyalkylene, alkylamide, (C₁₂-C₂₂)alkylamido(C₂-C₆)alkyl, (C₁₂-C₂₂)alkylacetate, and hydroxyalkyl radicals; and aromatic radicals such as aryl and alkylaryl; and X⁻ is chosen from halides, phosphates, acetates, lactates, (C₂-C₆) alkyl sulfates, and alkyl- or alkylaryl-sulfonates;
quaternary ammonium salts of imidazoline, for instance those of formula (B4) below: 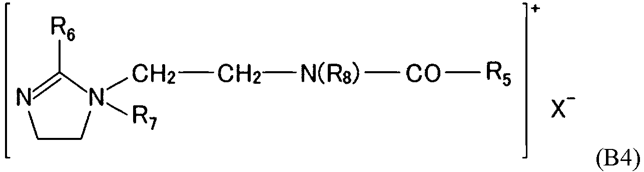 wherein:
   R₅ is chosen from alkenyl and alkyl radicals including from 8 to 30 carbon atoms, for example fatty acid derivatives of tallow or of coconut;
   R₆ is chosen from hydrogen, C₁-C₄ alkyl radicals, and alkenyl and alkyl radicals including from 8 to 30 carbon atoms;
   R₇ is chosen from C₁-C₄ alkyl radicals;
   R₈ is chosen from hydrogen and C₁-C₄ alkyl radicals; and
   X⁻ is chosen from halides, phosphates, acetates, lactates, alkyl sulfates, alkyl sulfonates, and alkylaryl sulfonates. In one embodiment, R₅ and R₆ are, for example, a mixture of radicals chosen from alkenyl and alkyl radicals including from 12 to 21 carbon atoms, such as fatty acid derivatives of tallow, R₇ is methyl, and R₈ is hydrogen. Examples of such products include, but are not limited to, Quaternium-27 (CTFA 1997) and Quaternium-83 (CTFA 1997), which are sold under the names "Rewoquat^{®}" W75, W90, W75PG and W75HPG by the company Witco;
   Di or tri quaternary ammonium salts of formula (B5): 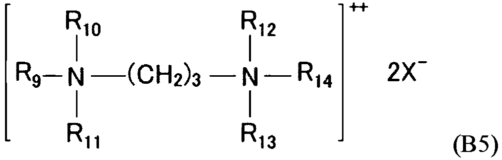 wherein:
      R₉ is chosen from aliphatic radicals including from 16 to 30 carbon atoms;
      R₁₀ is chosen from hydrogen or alkyl radicals including from 1 to 4 carbon atoms or a -(CH₂)₃ (R₁₆ₐ)(R₁₇ₐ)(R₁₈ₐ)N⁺X-₋ group;
      R₁₁, R₁₂, R₁₃, R₁₄, R₁₆ₐ, R₁₇ₐ, and R₁₈ₐ, which may be identical or different, are chosen from hydrogen and alkyl radicals including from 1 to 4 carbon atoms; and
      X⁻ is chosen from halides, acetates, phosphates, nitrates, ethyl sulfates, and methyl sulfates.

An example of one such diquaternary ammonium salt is FINQUAT CT-P of FINETEX (Quaternium-89) or FINQUAT CT (Quaternium-75);
and
quaternary ammonium salts including at least one ester function, such as those of formula (B6) below: 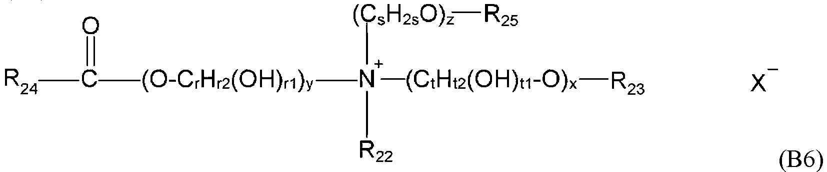 wherein:
R₂₂ is chosen from C₁-C₆ alkyl radicals and C₁-C₆ hydroxyalkyl and dihydroxyalkyl radicals;
R₂₃ is chosen from:
   the radical below: 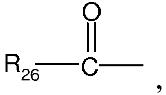
linear and branched, saturated and unsaturated C₁-C₂₂ hydrocarbon-based radicals R₂₇, and hydrogen,
R₂₅ is chosen from:
the radical below: 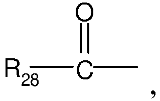
linear and branched, saturated and unsaturated C₁-C₆ hydrocarbon-based radicals R₂₉, and hydrogen,
R₂₄, R₂₆, and R₂₈, which may be identical or different, are chosen from linear and branched, saturated and unsaturated, C₇-C₂₁, hydrocarbon-based radicals;
r, s, and t, which may be identical or different, are chosen from integers ranging from 2 to 6;
each of r1 and t1, which may be identical or different, is 0 or 1, and r2+r1=2r and t1+2t=2t;
y is chosen from an integer ranging from 1 to 10;
x and z, which may be identical or different, are chosen from integers ranging from 0 to 10;
X⁻ is chosen from simple and complex, organic and inorganic anions; with the proviso that the sum x+y+z ranges from 1 to 15, that when x is 0, R₂₃ denotes R₂₇, and that when z is 0, R₂₅ denotes R₂₉. R₂₂ may be chosen from linear and branched alkyl radicals. In one embodiment, R₂₂ is chosen from linear alkyl radicals. In another embodiment, R₂₂ is chosen from methyl, ethyl, hydroxyethyl, and dihydroxypropyl radicals, for example methyl and ethyl radicals. In one embodiment, the sum x+y+z ranges from 1 to 10. When R₂₃ is a hydrocarbon-based radical R₂₇, it may be long and include from 12 to 22 carbon atoms, or short and include from 1 to 3 carbon atoms. When R₂₅ is a hydrocarbon-based radical R₂₉, it may include, for example, from 1 to 3 carbon atoms. By way of a non-limiting example, in one embodiment, R₂₄, R₂₆, and R₂₈, which may be identical or different, are chosen from linear and branched, saturated and unsaturated, C₁₁-C₂₁ hydrocarbon-based radicals, for example from linear and branched, saturated and unsaturated C₁₁-C₂₁ alkyl and alkenyl radicals. In another embodiment, x and z, which may be identical or different, are 0 or 1.

In one embodiment, y is equal to 1. In another embodiment, r, s, and t, which may be identical or different, are equal to 2 or 3, for example equal to 2. The anion X⁻ may be chosen from, for example, halides, such as chloride, bromide, and iodide; and C₁-C₄ alkyl sulfates, such as methyl sulfate. However, methane sulfonate, phosphate, nitrate, tosylate, an anion derived from an organic acid such as acetate and lactate, and any other anion that is compatible with the ammonium including an ester function, are other non-limiting examples of anions that may be used according to the present invention. In one embodiment, the anion X⁻ is chosen from chloride and methyl sulfate.

In another embodiment, the ammonium salts of formula (B6) may be used, wherein:
R₂₂ is chosen from methyl and ethyl radicals,
x and y are equal to 1;
z is equal to 0 or 1;
r, s, and t are equal to 2;
R₂₃ is chosen from:
   the radical below: 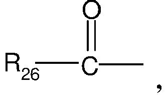
methyl, ethyl, and C₁₄-C₂₂ hydrocarbon-based radicals, and hydrogen;
R₂₅ is chosen from:
   the radical below: 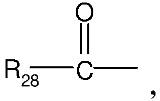
   and hydrogen;
   R₂₄, R₂₆, and R₂₈, which may be identical or different, are chosen from linear and branched, saturated and unsaturated, C₁₃-C₁₇ hydrocarbon-based radicals, for example from linear and branched, saturated and unsaturated, C₁₃-C₁₇ alkyl and alkenyl radicals.

In one embodiment, the hydrocarbon-based radicals are linear.

Non-limiting examples of compounds of formula (B6) that may be mentioned include salts, for example chloride and methyl sulfate, of diacyloxyethyl-dimethylammonium, of diacyloxyethyl-hydroxyethyl-methylammonium, of monoacyloxyethyl-dihydroxyethyl-methylammonium, of triacyloxyethyl-methylammonium, of monoacyloxyethyl-hydroxyethyl-dimethyl-ammonium, and mixtures thereof. In one embodiment, the acyl radicals may include from 14 to 18 carbon atoms, and may be derived, for example, from a plant oil, for instance palm oil and sunflower oil. When the compound includes several acyl radicals, these radicals may be identical or different.

These products may be obtained, for example, by direct esterification of optionally oxyalkylenated triethanolamine, triisopropanolamine, alkyldiethanolamine, or alkyldiisopropanolamine onto fatty acids or onto mixtures of fatty acids of plant or animal origin, or by transesterification of the methyl esters thereof. This esterification may be followed by a quaternization using an alkylating agent chosen from alkyl halides, for example methyl and ethyl halides; dialkyl sulfates, for example dimethyl and diethyl sulfates; methyl methanesulfonate; methyl para-toluenesulfonate; glycol chlorohydrin; and glycerol chlorohydrin.

Such compounds are sold, for example, under the names Dehyquart^{®} by the company Cognis, Stepanquat^{®} by the company Stepan, Noxamium^{®} by the company Ceca, and "Rewoquat^{®} WE 18" by the company Rewo-Goldschmidt.

Other non-limiting examples of ammonium salts that may be used in the composition according to the present invention include the ammonium salts including at least one ester function described in U.S. Pat. Nos. 4,874,554 and 4,137,180.

The quaternary ammonium salts mentioned above that may be used in the composition according to the present invention include, but are not limited to, those corresponding to formula (I), for example tetraalkylammonium chlorides, for instance dialkyldimethylammonium and alkyltrimethylammonium chlorides in which the alkyl radical includes from about 12 to 22 carbon atoms, such as behenyltrimethylammonium, distearyldimethylammonium, cetyltrimethylammonium and benzyldimethylstearylammonium chloride; palmitylamidopropyltrimethylammonium chloride; and stearamidopropyldimethyl(myristyl acetate)ammonium chloride, sold under the name "Ceraphyl^{®} 70" by the company Van Dyk.

According to one embodiment, the cationic surfactant that may be used in the composition according to the present invention is chosen from behenyltrimethylammonium chloride, cetyltrimethylammonium chloride, Quaternium-80, Quaternium-83, Quaternium-87, Quaternium-22, behenylamidopropyl-2,3-dihydroxypropyldimethylammonium chloride, palmitylamidopropyltrimethylammonium chloride, and stearamidopropyldimethylamine.

The amount of the (d) cationic surfactant(s) may be 0.1% by weight or more, preferably 0.5% by weight or more, and more preferably 1% by weight or more, relative to the total weight of the composition.

The amount of the (d) cationic surfactant(s) may be 15% by weight or less, preferably 10% by weight or less, and more preferably 5% by weight or less, relative to the total weight of the composition.

The amount of the (d) cationic surfactant(s) may be from 0.1% to 15% by weight, preferably from 0.5% to 10% by weight, and more preferably from 1% to 5% by weight, relative to the total weight of the composition.

### (Nonionic Surfactant)

The composition according to the present invention may comprise (e) at least one nonionic surfactant. If two or more (e) nonionic surfactants are used, they may be the same or different.

The nonionic surfactants are compounds well known in and of themselves (see, e.g., in this regard, "Handbook of Surfactants" by M. R. Porter, Blackie & Son publishers (Glasgow and London), 1991, pp. 116-178). Thus, they can, for example, be chosen from alcohols, alpha-diols, alkylphenols, and esters of fatty acids, these compounds being ethoxylated, propoxylated, or glycerolated and having at least one fatty chain comprising, for example, from 8 to 30 carbon atoms, it being possible for the number of ethylene oxide or propylene oxide groups to range from 2 to 50, and for the number of glycerol groups to range from 1 to 30. Maltose derivatives may also be mentioned. Non-limiting mention may also be made of copolymers of ethylene oxide and/or of propylene oxide; condensates of ethylene oxide and/or of propylene oxide with fatty alcohols; polyethoxylated fatty amides comprising, for example, from 2 to 30 mol of ethylene oxide; polyglycerolated fatty amides comprising, for example, from 1.5 to 5 glycerol groups, such as from 1.5 to 4; ethoxylated fatty acid esters of sorbitan comprising from 2 to 30 mol of ethylene oxide; ethoxylated oils of plant origin; fatty acid esters of sucrose; fatty acid esters of polyethylene glycol; polyethoxylated fatty acid mono or diesters of glycerol (C₆-C₂₄)alkylpolyglycosides; N-(C₆-C₂₄)alkylglucamine derivatives; amine oxides such as (C₁₀-C₁₄)alkylamine oxides or N-(C₁₀-C₁₄)acylaminopropylmorpholine oxides; silicone surfactants; and mixtures thereof.

The nonionic surfactants may preferably be chosen from monooxyalkylenated, polyoxyalkylenated, monoglycerolated, or polyglycerolated nonionic surfactants. The oxyalkylene units are more particularly oxyethylene or oxypropylene units, or a combination thereof, and are preferably oxyethylene units.

Examples of monooxyalkylenated or polyoxyalkylenated nonionic surfactants that may be mentioned include:
monooxyalkylenated or polyoxyalkylenated (C₈-C₂₄)alkylphenols,
saturated or unsaturated, linear or branched, monooxyalkylenated or polyoxyalkylenated C₈-C₃₀ alcohols,
saturated or unsaturated, linear or branched, monooxyalkylenated or polyoxyalkylenated C₈-C₃₀ amides,
esters of saturated or unsaturated, linear or branched, C₈-C₃₀ acids and of monoalkyleneglycols or polyalkyleneglycols,
monooxyalkylenated or polyoxyalkylenated esters of saturated or unsaturated, linear or branched, C₈-C₃₀ acids, and of sorbitol,
saturated or unsaturated, monooxyalkylenated or polyoxyalkylenated plant oils, and
condensates of ethylene oxide and/or of propylene oxide, inter alia, alone or as mixtures.

The surfactants preferably contain a number of moles of ethylene oxide and/or of propylene oxide of between 1 and 100, preferably between 1 and 50, and more preferably between 1 and 20.

According to one of the embodiments of the present invention, the monooxyalkylenated nonionic surfactants may be chosen from monooxyethylenated fatty alcohol (ether of ethyleneglycol and fatty alcohol), monooxyethylenated fatty ester (ester of ethyleneglycol and fatty acid), and mixtures thereof.

Examples of monooxyalkylenated fatty ester that may be mentioned include glycol distearate.

According to one of the embodiments of the present invention, the polyoxyalkylenated nonionic surfactants may be chosen from polyoxyethylenated fatty alcohol (ether of polyethyleneglycol and fatty alcohol), polyoxyethylenated fatty ester (ester of polyethyleneglycol and fatty acid), and mixtures thereof.

Examples of polyoxyethylenated saturated fatty alcohol (or C₈-C₃₀ alcohols) that may be mentioned include the adducts of ethylene oxide with lauryl alcohol, especially those containing from 2 to 20 oxyethylene units and more particularly those containing from 2 to 10 oxyethylene units (Laureth-2 to Laureth-20, as the CTFA names); the adducts of ethylene oxide with behenyl alcohol, especially those containing from 2 to 20 oxyethylene units (Beheneth-2 to Beheneth-20, as the CTFA names); the adducts of ethylene oxide with cetearyl alcohol (mixture of cetyl alcohol and stearyl alcohol), especially those containing from 2 to 20 oxyethylene units (Ceteareth-2 to Ceteareth-20, as the CTFA names); the adducts of ethylene oxide with cetyl alcohol, especially those containing from 2 to 20 oxyethylene units (Ceteth-2 to Ceteth-20, as the CTFA names); the adducts of ethylene oxide with stearyl alcohol, especially those containing from 2 to 20 oxyethylene units (Steareth-2 to Steareth-20, as the CTFA names); the adducts of ethylene oxide with isostearyl alcohol, especially those containing from 2 to 20 oxyethylene units (Isosteareth-2 to Isosteareth-20, as the CTFA names); and mixtures thereof.

Examples of polyoxyethylenated unsaturated fatty alcohol (or C₈-C₃₀ alcohols) that may be mentioned include the adducts of ethylene oxide with oleyl alcohol, especially those containing from 2 to 20 oxyethylene units and more particularly those containing from 2 to 10 oxyethylene units (Oleth-2 to Oleth-20, as the CTFA names); and mixtures thereof.

As examples of monoglycerolated or polyglycerolated nonionic surfactants, monoglycerolated or polyglycerolated C₈-C₄₀ alcohols are preferably used.

In particular, the monoglycerolated or polyglycerolated C₈-C₄₀ alcohols correspond to the following formula:

RO-[CH₂-CH(CH₂OH)-O]ₘ-H or RO-[CH(CH₂OH)-CH₂O]ₘ-H

in which R represents a linear or branched C₈-C₄₀ and preferably C₈-C₃₀ alkyl or alkenyl radical, and m represents a number ranging from 1 to 30 and preferably from 1.5 to 10.

As examples of compounds that are suitable in the context of the present invention, mention may be made of lauryl alcohol containing 4 mol of glycerol (INCI name: Polyglyceryl-4 Lauryl Ether), lauryl alcohol containing 1.5 mol of glycerol, oleyl alcohol containing 4 mol of glycerol (INCI name: Polyglyceryl-4 Oleyl Ether), oleyl alcohol containing 2 mol of glycerol (INCI name: Polyglyceryl-2 Oleyl Ether), cetearyl alcohol containing 2 mol of glycerol, cetearyl alcohol containing 6 mol of glycerol, oleocetyl alcohol containing 6 mol of glycerol, and octadecanol containing 6 mol of glycerol.

The alcohol may represent a mixture of alcohols in the same way that the value of m represents a statistical value, which means that, in a commercial product, several species of polyglycerolated fatty alcohol may coexist in the form of a mixture.

Among the monoglycerolated or polyglycerolated alcohols, it is preferable to use a C₈/C₁₀ alcohol containing 1 mol of glycerol, a C₁₀/C₁₂ alcohol containing 1 mol of glycerol, and a C₁₂ alcohol containing 1.5 mol of glycerol.

The monoglycerolated or polyglycerolated C₈-C₄₀ fatty esters may correspond to the following formula:

R'O-[CH₂-CH(CH₂OR"')-O]ₘ-R" or R'O-[CH(CH₂OR"')-CH₂O]ₘ-R"

in which each of R', R", and R"' independently represents a hydrogen atom, or a linear or branched C₈-C₄₀ and preferably C₈-C₃₀ alkyl-CO- or alkenyl-CO-radical, with the proviso that at least one of R', R", and R‴ is not a hydrogen atom, and m represents a number ranging from 1 to 30 and preferably from 1.5 to 10.

Examples of polyoxyethylenated fatty esters that may be mentioned include the adducts of ethylene oxide with esters of lauric acid, palmitic acid, stearic acid or behenic acid, and mixtures thereof, especially those containing from 2 to 20 oxyethylene units, such as PEG-2 to PEG-20 laurate (CTFA names: PEG-2 laurate to PEG-20 laurate); PEG-2 to PEG-20 palmitate (CTFA names: PEG-2 palmitate to PEG-20 palmitate); PEG-2 to PEG-20 stearate (CTFA names: PEG-2 stearate to PEG-20 stearate); PEG-2 to PEG-20 palmitostearate; PEG-2 to PEG-20 behenate (CTFA names: PEG-2 behenate to PEG-20 behenate); and mixtures thereof.

The polyoxyethylenated fatty esters may also be selected from diesters of polyethyleneglycol and fatty acids, such as saturated or unsaturated, linear or branched, C₈-C₃₀ acids, which may have one or more substituents such as a hydroxyl group and hydroxyl groups. The fatty acids may be in the form of a polymer of fatty acids each of which has one or more hydroxyl groups. Such a polymer may be formed by the esterification of the carboxyl group of one fatty acid having one or more hydroxyl groups and the hydroxyl group of another fatty acid having one or more hydroxyl groups. Examples of such a polymer include polyhydroxystearate. Thus, as the polyoxyethylenated fatty ester, mention may be made of PEG-30 dipolyhydroxystearate.

According to one of the embodiments of the present invention, the nonionic surfactant may be selected from esters of polyols with fatty acids with a saturated or unsaturated chain containing for example from 8 to 24 carbon atoms, preferably 12 to 22 carbon atoms, and polyoxyalkylenated derivatives thereof, preferably containing from 10 to 200, and more preferably from 10 to 100 oxyalkylene units, such as glyceryl esters of a C₈-C₂₄, preferably C₁₂-C₂₂, fatty acid or acids, and polyoxyalkylenated derivatives thereof, preferably containing from 10 to 200, and more preferably from 10 to 100 oxyalkylene units; sorbitol esters of a C₈-C₂₄, preferably C₁₂-C₂₂, fatty acid or acids, and polyoxyalkylenated derivatives thereof, preferably containing from 10 to 200, and more preferably from 10 to 100 oxyalkylene units; sugar (sucrose, maltose, glucose, fructose, and/or alkylglycose) esters of a C₈-C₂₄, preferably C₁₂-C₂₂, fatty acid or acids, and polyoxyalkylenated derivatives thereof, preferably containing from 10 to 200, and more preferably from 10 to 100 oxyalkylene units; ethers of fatty alcohols; ethers of sugar and a C₈-C₂₄, preferably C₁₂-C₂₂, fatty alcohol or alcohols; and mixtures thereof.

As glyceryl esters of fatty acids, glyceryl stearate (glyceryl mono-, di-, and/or tristearate) (CTFA name: glyceryl stearate), glyceryl laurate or glyceryl ricinoleate, and mixtures thereof can be cited, and as polyoxyalkylenated derivatives thereof, mono-, di-, or triester of fatty acids with a polyoxyalkylenated glycerol (mono-, di-, or triester of fatty acids with a polyalkylene glycol ether of glycerol), preferably polyoxyethylenated glyceryl stearate (mono-, di-, and/or tristearate) such as PEG-20 glyceryl stearate (mono-, di-, and/or tristearate) and polyoxyethylenated glyceryl cocoate (mono-, di-, and/or tristearate) such as PEG-7 glyceryl cocoate can be cited.

Mixtures of these surfactants, such as for example the product containing glyceryl stearate and PEG-100 stearate, marketed under the name ARLACEL 165 by Uniqema, and the product containing glyceryl stearate (glyceryl mono- and distearate) and potassium stearate marketed under the name TEGIN by Goldschmidt (CTFA name: glyceryl stearate SE), can also be used.

The sorbitol esters of C₈-C₂₄ fatty acids and polyoxyalkylenated derivatives thereof can be selected from sorbitan palmitate, sorbitan isostearate, sorbitan trioleate, sorbitan sesquioleate and esters of fatty acids and alkoxylated sorbitan containing for example from 20 to 100 EO, such as for example sorbitan monostearate (CTFA name: sorbitan stearate), sold by the company ICI under the name Span 60, sorbitan monopalmitate (CTFA name: sorbitan palmitate), sold by the company ICI under the name Span 40, and sorbitan tristearate 20 EO (CTFA name: polysorbate 65), sold by the company ICI under the name Tween 65, polyethylene sorbitan trioleate (polysorbate 85), or the compounds marketed under the trade names Tween 20 (polysorbate 20) or Tween 80 (polysorbate 80).

As esters of fatty acids and glucose or alkylglucose, glucose palmitate, alkylglucose sesquistearates such as methylglucose sesquistearate, alkylglucose palmitates such as methylglucose or ethylglucose palmitate, methylglucoside fatty esters, the diester of methylglucoside and oleic acid (CTFA name: Methyl glucose dioleate), the mixed ester of methylglucoside and the mixture of oleic acid/hydroxystearic acid (CTFA name: Methyl glucose dioleate/hydroxystearate), the ester of methylglucoside and isostearic acid (CTFA name: Methyl glucose isostearate), the ester of methylglucoside and lauric acid (CTFA name: Methyl glucose laurate), the mixture of monoester and diester of methylglucoside and isostearic acid (CTFA name: Methyl glucose sesqui-isostearate), the mixture of monoester and diester of methylglucoside and stearic acid (CTFA name: Methyl glucose sesquistearate) and in particular the product marketed under the name Glucate SS by AMERCHOL, and mixtures thereof can be cited.

As ethoxylated ethers of fatty acids and glucose or alkylglucose, ethoxylated ethers of fatty acids and methylglucose, and in particular the polyethylene glycol ether of the diester of methylglucose and stearic acid with about 20 moles of ethylene oxide (CTFA name: PEG-20 methyl glucose distearate) such as the product marketed under the name Glucam E-20 distearate by AMERCHOL, the polyethylene glycol ether of the mixture of monoester and diester of methyl-glucose and stearic acid with about 20 moles of ethylene oxide (CTFA name: PEG-20 methyl glucose sesquistearate) and in particular the product marketed under the name Glucamate SSE-20 by AMERCHOL and that marketed under the name Grillocose PSE-20 by GOLDSCHMIDT, and mixtures thereof, can for example be cited.

As sucrose esters, saccharose palmito-stearate, saccharose stearate, and saccharose monolaurate can for example be cited.

As sugar ethers, alkylpolyglucosides can be used, and for example decylglucoside such as the product marketed under the name MYDOL 10 by Kao Chemicals, the product marketed under the name PLANTAREN 2000 by Henkel, and the product marketed under the name ORAMIX NS 10 by Seppic, caprylyl/capryl glucoside such as the product marketed under the name ORAMIX CG 110 by Seppic or under the name LUTENSOL GD 70 by BASF, laurylglucoside such as the products marketed under the names PLANTAREN 1200 N and PLANTACARE 1200 by Henkel, coco-glucoside such as the product marketed under the name PLANTACARE 818/UP by Henkel, cetostearyl glucoside possibly mixed with cetostearyl alcohol, marketed for example under the name MONTANOV 68 by Seppic, under the name TEGO-CARE CG90 by Goldschmidt and under the name EMULGADE KE3302 by Henkel, arachidyl glucoside, for example in the form of the mixture of arachidyl and behenyl alcohols and arachidyl glucoside marketed under the name MONTANOV 202 by Seppic, cocoylethylglucoside, for example in the form of the mixture (35/65) with cetyl and stearyl alcohols, marketed under the name MONTANOV 82 by Seppic, and mixtures thereof can in particular be cited.

Mixtures of glycerides of alkoxylated plant oils such as mixtures of ethoxylated (200 EO) palm and copra (7 EO) glycerides can also be cited.

The nonionic surfactant according to the present invention may preferably contain alkenyl or a branched C₁₂-C₂₂ acyl chain such as an oleyl or isostearyl group. More preferably, the nonionic surfactant according to the present invention is PEG-20 glyceryl triisostearate.

According to one of the embodiments of the present invention, the nonionic surfactant may be selected from copolymers of ethylene oxide and of propylene oxide, in particular copolymers of the following formula:

HO(C₂H₄O)ₐ(C₃H₆O)_{b}(C₂H₄O)_{c}H

in which a, b, and c are integers such that a+c ranges from 2 to 100 and b ranges from 14 to 60, and mixtures thereof.

According to one of the embodiments of the present invention, the nonionic surfactant may be selected from silicone surfactants. Non-limiting mention may be made of those disclosed in documents US-A-5364633 and US-A-5411744.

The silicone surfactant may preferably be a compound of formula (I): 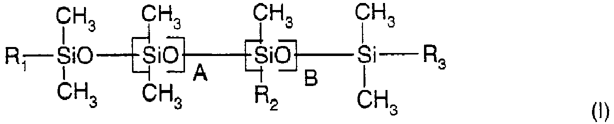 in which:
R₁, R₂, and R₃, independently of each other, represent a C₁-C₆ alkyl radical or a radical - (CH₂)ₓ-(OCH₂CH₂)_{y}-(OCH₂CH₂CH₂)_{z}-OR₄, at least one radical R₁, R₂, or R₃ not being an alkyl radical; R₄ being a hydrogen, an alkyl radical, or an acyl radical;
A is an integer ranging from 0 to 200;
B is an integer ranging from 0 to 50; with the proviso that A and B are not simultaneously equal to zero;
x is an integer ranging from 1 to 6;
y is an integer ranging from 1 to 30;
z is an integer ranging from 0 to 5.

According to one preferred embodiment of the present invention, in the compound of formula (I), the alkyl radical is a methyl radical, x is an integer ranging from 2 to 6, and y is an integer ranging from 4 to 30.

As examples of silicone surfactants of formula (I), mention may be made of the compounds of formula (II): 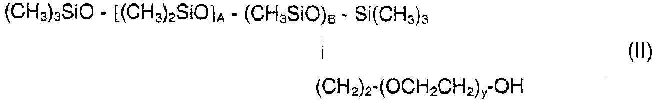 in which A is an integer ranging from 20 to 105, B is an integer ranging from 2 to 10, and y is an integer ranging from 10 to 20.

As examples of silicone surfactants of formula (I), mention may also be made of the compounds of formula (III):

H-(OCH₂CH₂)_{y}-(CH₂)₃-[(CH₃)₂SiO]_{A'}-(CH₂)₃-(OCH₂CH₂)_{y}-OH (III)

in which A' and y are integers ranging from 10 to 20.

Compounds of the present invention which may be used are those sold by the company Dow Corning under the names DC 5329, DC 7439-146, DC 2-5695, and Q4-3667. The compounds DC 5329, DC 7439-146, and DC 2-5695 are compounds of formula (II) in which, respectively, A is 22, B is 2, and y is 12; A is 103, B is 10, and y is 12; A is 27, B is 3, and y is 12.

The compound Q4-3667 is a compound of formula (III) in which A is 15 and y is 13.

The amount of the (e) nonionic surfactant(s) in the composition according to the present invention may be 0.1% by weight or more, preferably 0.5% by weight or more, and more preferably 1.6% by weight or more, relative to the total weight of the composition.

The amount of the (e) nonionic surfactant(s) in the composition according to the present invention may be 15% by weight or less, preferably 10% by weight or less, and more preferably 5% by weight or less, relative to the total weight of the composition.

The amount of the (e) nonionic surfactant(s) in the composition according to the present invention may be from 0.1% to 15% by weight, preferably from 0.5% to 10% by weight, and more preferably from 1.6% to 5% by weight, relative to the total weight of the composition.

### (Amino Acid, Derivative Thereof and Salts Thereof)

The composition according to the present invention may comprise at least one compound selected from amino acids, derivatives thereof, and salts thereof (hereafter, may be referred to as (f) compound). If two or more (f) compounds are used, they may be the same or different.

In one embodiment, the (f) compound is selected from amino acids.

The amino acid has at least one amino group and at least one carboxyl group.

The amino group may be a primary amino group, a secondary amino group or a tertiary amino group, preferably a primary amino group or a secondary amino group, and more preferably a secondary amino group.

It is preferable that the molecular weight of the amino acid be less than 1,000, more preferably less than 500, and even more preferably less than 200. Thus, it is preferable that the amino acid not be a polymer. In other words, it is preferable that the amino acid be a non-polymeric amino acid.

The amino acid may be selected from acidic amino acids, basic amino acids and neutral amino acids. The acidic amino acids typically have one amino group and two carboxyl groups. The basic amino acids typically have two amino groups and one carboxyl group. The number of amino group(s) and the number of carboxyl group(s) in the neutral amino groups are the same.

The amino acid may be in the D- or L-form.

The amino acid may be hydrophilic or hydrophobic. A hydrophilic amino acid is preferable.

The amino acid may be selected from α-amino acids, β-amino acids, γ-amino acids and δ-amino acids.

It is preferable that the amino acid be selected from an α-amino acid in which an amino group is bonded to the carbon atom to which a carboxyl group is bonded.

The α-amino acid may be selected from non-cyclic α-amino acids and cyclic α-amino acids.

The non-cyclic α-amino acid may be selected from the group consisting of alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine and valine.

The cyclic α-amino acid may be selected from non-aromatic cyclic α-amino acids such as pyrrolidone carboxylic acid (pyroglutamic acid or pidolic acid). Pyrrolidone carboxylic acid can be formed by intramolecular condensation with the amino group and the carboxyl group of glutamic acid.

In one embodiment, the (f) compound is selected from the derivatives of amino acids.

The derivatives of amino acids (amino acid derivatives) may be selected from amino acids in which the hydrogen atom on the nitrogen atom of the amino group in the amino acids is substituted with at least one substituent.

As the substituent, mention may be made of, for example, an alkyl group, acyl group, an alkenyl group, an alkoxyl group and an alkoxycarbonyl group.

The alkyl group may be a linear, branched or cyclic alkyl group. The alkyl group may be a linear or branched C₁-C₆ alkyl group, preferably C₁-C₄ alkyl group, such as a methyl group, an ethyl group, a propyl group, an i-propyl group and a butyl group. On the other hand, the alkyl group may be a cyclic C₃-C₆ alkyl group, such as a cyclopentyl group and a cyclohexyl group.

The acyl group may be a C₁-C₆ acyl group such as a formyl group and an acetyl group.

The alkenyl group may be a C₂-C₆ alkenyl group such as a vinyl group, an allyl group, a butylene group, a pentenyl group and a hexenyl group.

The alkoxy group may be a C₁-C₆ alkoxy group such as a methoxy group, an ethoxy group and a propoxy group.

The alkoxycarbonyl group may be a C₁-C₆ alkoxycarbonyl group such as a methoxycarbonyl group, an ethoxycarbonyl group, and a propoxycarbonyl group.

The above substituent may be further substituted with at least one group such as a halogen atom, an amino group, a nitro group, a cyano group, a hydroxyl group and an aromatic group such as a phenyl group.

In one embodiment, the (f) compound is selected from the salts of amino acids or the salts of amino acid derivatives.

The type of the salts of amino acids or the salts of amino acid derivatives is not limited. The salts may be acid salts or basic salts. As acid salts, mention may be made of, for example, inorganic acid salts such as hydrochloride, sulfates, nitrates, and phosphates, and organic acid salts such as citrates, oxalates, acetates, formats, maleates, and tartrates. As basic salts, mention may be made of, for example, inorganic base salts such as sodium salt, potassium salt, calcium salt, magnesium salt, copper salt, zinc salt, aluminum salt and ammonium salts, and organic base salts such as triethylammonium salts, triethanolammonium salts, pyridinium salts and diisopropyl ammonium salts. Sodium salt is preferable.

The amount of the (f) compound(s) in the composition according to the present invention may be 1% by weight or more, preferably 5% by weight or more, and more preferably 10% by weight or more, relative to the total weight of the composition.

The amount of the (f) compound(s) in the composition according to the present invention may be 30% by weight or less, preferably 25% by weight or less, and more preferably 20% by weight or less, relative to the total weight of the composition.

The amount of the (f) compound(s) in the composition according to the present invention may be from 1% to 30% by weight, preferably from 5% to 25% by weight, and more preferably from 10% to 20% by weight, relative to the total weight of the composition.

### (Optional Components)

The composition according to the present invention may comprise, in addition to the aforementioned components, optional components typically employed in cosmetics, specifically, anionic or amphoteric surfactants, oils, dyes, fillers, polyols such as glycols and glycerol, hydrophilic or lipophilic thickeners, UV filters, natural extracts derived from animals or vegetables, preservatives, and the like, within a range which does not impair the effects of the present invention.

The composition according to the present invention may comprise the above optional component(s) in an amount of from 0.001% to 30% by weight, preferably from 0.01% to 20% by weight, and more preferably from 0.1% to 10% by weight, relative to the total weight of the composition.

The composition according to the present invention includes only a limited amount of silicone(s). More specifically, the amount of silicone(s) in the composition according to the present invention is 1% by weight or less, relative to the total weight of the composition. It is preferable that the composition according to the present invention include no silicone.

### (Preparation)

The composition according to the present invention can be prepared by mixing the above essential and optional ingredients in accordance with any of the processes which are well known to those skilled in the art.

The composition according to the present invention can be in the form of a fluid, preferably a liquid or a paste, and more preferably a liquid.

### (Applications)

The composition according to the present invention can be used for treating, e.g., caring for or conditioning, keratin fibers.

The composition according to the present invention may be a cosmetic composition, preferably a rinse-off type cosmetic composition, and more preferably a rinse-off type hair cosmetic composition

For example, the composition according to the present invention may be used in hair care cosmetic products such as shampoos, conditioners and the like.

### [Cosmetic Process and Use]

The present invention also relates to a cosmetic process for caring for or conditioning keratin fibers, preferably hair, comprising the step of:
applying onto the keratin fibers the composition according to the present invention.

The present invention may also relate to a process for enhancing or improving the smoothness of keratin fibers, preferably hair, and more preferably wet hair, comprising applying onto the keratin fibers the composition according to the present invention.

The applying step can be performed by any conventional means such as an applicator, e.g., a brush.

### EXAMPLES

The present invention will be described in a more detailed manner by way of examples. However, these examples should not be construed as limiting the scope of the present invention.

### Examples 1 and 2 and Comparative Examples 1 and 2

### [Preparation]

Each of the compositions according to Examples 1 and 2 (Ex. 1 and Ex. 2) and Comparative Examples 1 and 2 (Comp. Ex. 1 and Comp. Ex. 2) was prepared by mixing the ingredients shown in Table 1. The numerical values for the amounts of the ingredients shown in Table 1 are all based on "% by weight" as active materials, relative to the total weight of the composition.

**Table 1**

| | | Ex. 1 | Ex. 2 | Comp. Ex. 1 | Comp. Ex. 2 |
|---|---|---|---|---|---|
| Glycerin | | 15 | 15 | 15 | 15 |
| Glycine | | 5 | 5 | 5 | 5 |
| Propylene Glycol | | 5 | 5 | 5 | 5 |
| Polysorbate 20 | | 0.8 | 0.8 | 0.8 | 0.8 |
| Polysorbate 80 | | 0.8 | 0.8 | 0.8 | 0.8 |
| Citric Acid | | 2.95 | 2.95 | 2.95 | 2.95 |
| Hydroxypropyl Trimonium Hvdrolvzed Wheat Protein | | 0.91 | 0.39 | 0.13 | - |
| Cetrimonium Chloride | | 0.5 | 0.5 | 0.5 | 0.5 |
| Quaternium-80 | | 0.38 | 0.38 | 0.38 | 0.38 |
| Sodium Hydroxide | | qs pH 4.0 | qs pH 4.0 | qs pH 4.0 | qs pH 4.0 |
| Water | | qsp 100 | qsp 100 | qsp 100 | qsp 100 |
| Smoothness (COF) | | 0.201 | 0.204 | 0.214 | 0.230 |
| Sensory Assessments | Smoothness | 5 | 5 | 3.7 | 4 |
| | Combing | 5 | 5 | 4 | 3.7 |
| | Softness | 4 | 4 | 4 | 4 |

### [Evaluations]

### (Smoothness (COF))

Three hair swatches (1 g, 27 cm) were prepared for each experiment. Each of the hair swatches was washed with a clarifying shampoo once. After shampooing, the hair swatch was rinsed with water. 0.4 g of each of the compositions according to Examples 1 and 2 and Comparative Examples 1 and 2 was applied onto each hair swatch, and left for 5 minutes. The hair swatch was then rinsed off under running water, and then dried naturally under ambient conditions to obtain a treated hair swatch.

The treated hair swatch was placed on a plate, and its root side was fixed on the plate with a hair clip. The smoothness of the treated hair swatch was evaluated by scanning the hair swatch from root to tip with a sensor (Handy Rub Tester (type TL701) from Trinity Lab) and measuring COF (Coefficient of Friction). The measurement was carried out 3 times for a single treated hair swatch. The same procedure was conducted with two more hair swatches to obtain 9 results in total for one composition, and the mean values were calculated for each composition. A lower score indicates that a better smoothness effect can be exerted.

The COF of the hair swatch itself was 0.233.

The results are shown on the line labelled "Smoothness (COF)" in Table 1. The smaller the COF is, the greater the smoothness is.

### {Sensory Assessments}

Three hair swatches (2.7 g, 27 cm) were prepared for each experiment. Each of the hair swatches was washed with a clarifying shampoo once. After shampooing, the hair swatch was rinsed with water. 1.1 g of each of the compositions according to Examples 1 and 2 and Comparative Examples 1 and 2 was applied onto each hair swatch. The hair swatch was rinsed off under running water, and then dried naturally under ambient conditions to obtain a treated hair swatch.

The smoothness, combing, and softness, after the application of each of the compositions according to Examples 1 and 2 and Comparative Examples 1 and 2 onto the hair swatch, were assessed by 3 panelists in accordance with the following criteria. The benchmark means the assessment result for the hair swatch itself, i.e., without the application of any composition.
5: Much better than benchmark
4: Better than benchmark
3: Parity to Benchmark
2: Worse than benchmark
1: Much worse than benchmark

The scores thus obtained were averaged. The results are shown on the line labelled "Sensory Assessments" in Table 1.

### (Results)

As can be seen from the results shown in Table 1, the compositions according to Examples 1 and 2, each of which comprises both citric acid and hydrolyzed wheat protein in an amount of 0.2% by weight or more relative to the total weight of the composition, were able to provide hair with good smoothness. It should be noted that a COF value of less than 0.210 is a threshold to consider to provide good smoothness.

The composition according to Comparative Example 1, which comprises citric acid and hydrolyzed wheat protein in an amount of 0.2% by weight relative to the total weight of the composition was able to provide hair with some smoothness, but the degree of the smoothness was insufficient to be considered as being good.

The composition according to Comparative Example 2, which comprises citric acid but no hydrolyzed wheat protein was able to provide hair with only very slight smoothness.

Furthermore, the compositions according to Examples 1 and 2 were able to provide hair with better cosmetic effects in terms of all of smoothness, combing and softness, than the compositions according to Comparative Examples 1 and 2.

### Example 3

The composition according to Example 3 (Ex. 3) is prepared by mixing the ingredients shown in Table 2. The numerical values for the amounts of the ingredients shown in Table 1 are all based on "% by weight" as active materials, relative to the total weight of the composition.

**Table 2**

| | Ex. 3 |
|---|---|
| Glycerin | 15 |
| Glycine | 5 |
| Propylene Glycol | 5 |
| Polysorbate 20 | 0.8 |
| Polysorbate 80 | 0.8 |
| Tartaric Acid | 5 |
| Hydroxypropyl Trimonium Hydrolyzed Wheat Protein | 0.91 |
| Cetrimonium Chloride | 0.5 |
| Quaternium-80 | 0.38 |
| Sodium Hydroxide | qs pH 4.0 |
| Water | qsp 100 |

The composition according to Example 3 can provide hair with good smoothness.

Furthermore, the composition according to Example 3 can provide hair with good cosmetic effects in terms of all of smoothness, combing and softness.

## Claims

1. A composition for treating keratin fibers, preferably hair, comprising:
(a) at least one α-hydroxy acid or salt thereof;
(b) at least one cationic polyamino acid comprising at least one quaternary ammonium group and being derived from plant proteins; and
(c) water,
wherein
the amount of the (a) α-hydroxy acid or salt thereof in the composition is 1% by weight or more, relative to the total weight of the composition,
the amount of the (b) cationic polyamino acid(s) is 0.2% by weight or more, preferably 0.3% by weight or more, more preferably 0.5% by weight or more, and even more preferably 0.8% by weight or more, relative to the total weight of the composition, and the amount of silicone (s) in the composition is 1% by weight or less, relative to the total weight of the composition.

2. The composition according to claim 1, wherein the (a) α-hydroxy acid has two or more carboxylic groups.

3. The composition according to claim 1 or 2, wherein the (a) α-hydroxy acid is selected from the group consisting of glycolic acid, lactic acid, malic acid, citric acid, tartaric acid, mandelic acid, gluconic acid, mucic acid, and a mixture thereof, and more preferably from the group consisting of citric acid, tartaric acid, and a mixture thereof.

4. The composition according to any one of claims 1 to 3, wherein the amount of the (a) α-hydroxy acid(s) or salt thereof in the composition is from 1% to 10% by weight, relative to the total weight of the composition.

5. The composition according to any one of claims 1 to 4, wherein the (b) cationic polyamino acid is selected from the group consisting of hydroxypropyl trimonium hydrolyzed wheat protein, steardimonium hydroxypropyl hydrolyzed wheat protein, cocodimonium hydroxypropyl hydrolyzed wheat protein, hydroxypropyltrimonium hydrolyzed conchiolin protein, steardimonium hydroxypropyl hydrolyzed soy protein, hydroxypropyltrimonium hydrolyzed soy protein, cocodimonium hydroxypropyl hydrolyzed soy protein, and a mixture thereof.

6. The composition according to any one of claims 1 to 5, wherein the amount of the (b) cationic polyamino acid(s) in the composition is 20% by weight or less, preferably 15% by weight or less, more preferably 10% by weight or less, and even more preferably 5% by weight or less, relative to the total weight of the composition.

7. The composition according to any one of claims 1 to 6, wherein the amount of the (c) water in the composition is from 50% to 80% by weight, preferably from 55% to 75% by weight, and more preferably from 60% to 70% by weight, relative to the total weight of the composition.

8. The composition according to any one of claims 1 to 7, wherein the pH of the composition is less than 7, preferably less than 6, and more preferably less than 5.

9. The composition according to any one of claims 1 to 8, further comprising (d) at least one cationic surfactant which is different from the (b) cationic polyamino acid.

10. The composition according to any one of claims 1 to 9, further comprising (e) at least one nonionic surfactant.

11. The composition according to any one of claims 1 to 10, wherein the composition is a cosmetic composition, preferably a rinse-off type cosmetic composition, and more preferably a rinse-off type hair cosmetic composition.

12. A cosmetic process for caring for or conditioning keratin fibers, preferably hair, comprising the step of:
applying onto the keratin fibers the composition according to any one of claims 1 to 11.

## Patentansprüche

1. Zusammensetzung zum Behandeln von Keratinfasern, vorzugsweise Haar, umfassend:
(a) mindestens eine α-Hydroxysäure oder ein Salz davon;
(b) mindestens eine kationische Polyaminosäure, umfassend mindestens eine quaternäre Ammoniumgruppe und die von Pflanzenproteinen abgeleitet ist; und
(c) Wasser,
wobei
die Menge der (a) α-Hydroxysäure oder des Salzes davon in der Zusammensetzung 1 Gewichts-% oder mehr, bezogen auf das Gesamtgewicht der Zusammensetzung, ist,
die Menge der (b) kationischen Polyaminosäure(n) 0,2 Gewichts-% oder mehr, vorzugsweise 0,3 Gewichts-% oder mehr, bevorzugter 0,5 Gewichts-% oder mehr und noch bevorzugter 0,8 Gewichts-% oder mehr, bezogen auf das Gesamtgewicht der Zusammensetzung, ist und die Menge an Silikon(en) in der Zusammensetzung 1 Gewichts-% oder weniger, bezogen auf das Gesamtgewicht der Zusammensetzung, ist.

2. Zusammensetzung nach Anspruch 1, wobei die (a) α-Hydroxysäure zwei oder mehrere Carboxylgruppen aufweist.

3. Zusammensetzung nach Anspruch 1 oder 2, wobei die (a) α-Hydroxysäure ausgewählt ist aus der Gruppe, bestehend aus Glykolsäure, Milchsäure, Äpfelsäure, Zitronensäure, Weinsäure, Mandelsäure, Gluconsäure, Schleimsäure und einem Gemisch davon, und vorzugsweise aus der Gruppe, bestehend aus Zitronensäure, Weinsäure und einem Gemisch davon.

4. Zusammensetzung nach einem der Ansprüche 1 bis 3, wobei die Menge der (a) α-Hydroxysäure oder des Salzes davon in der Zusammensetzung von 1 bis 10 Gewichts-%, bezogen auf das Gesamtgewicht der Zusammensetzung, ist.

5. Zusammensetzung nach einem der Ansprüche 1 bis 4, wobei die kationische Polyaminosäure (b) ausgewählt ist aus der Gruppe bestehend aus Hydroxypropyltrimonium-hydrolysiertem Weizenprotein, Steardimoniumhydroxypropyl-hydrolysiertem Weizenprotein, Cocodimoniumhydroxypropyl-hydrolysiertem Weizenprotein, Hydroxypropyltrimonium-hydrolysiertem Conchiolinprotein, Steardimoniumhydroxypropyl-hydrolysiertem Sojaprotein, Hydroxypropyltrimonium-hydrolysiertem Sojaprotein, Cocodimoniumhydroxypropyl-hydrolysiertem Sojaprotein und einem Gemisch davon.

6. Zusammensetzung nach einem der Ansprüche 1 bis 5, wobei die Menge der (b) kationischen Polyaminosäure(n) in der Zusammensetzung 20 Gewichts-% oder weniger, vorzugsweise 15 Gewichts-% oder weniger, bevorzugter 10 Gewichts-% oder weniger und noch bevorzugter 5 Gewichts-% oder weniger, bezogen auf das Gesamtgewicht der Zusammensetzung, ist.

7. Zusammensetzung nach einem der Ansprüche 1 bis 6, wobei die Menge des (c) Wassers in der Zusammensetzung von 50 bis 80 Gewichts-%, vorzugsweise von 55 bis 75 Gewichts-% und bevorzugter von 60 bis 70 Gewichts-%, bezogen auf das Gesamtgewicht der Zusammensetzung, ist.

8. Zusammensetzung nach einem der Ansprüche 1 bis 7, wobei der pH der Zusammensetzung weniger als 7, vorzugsweise weniger als 6, und bevorzugter weniger als 5 ist.

9. Zusammensetzung nach einem der Ansprüche 1 bis 8, ferner umfassend (d) mindestens ein kationisches Tensid, das sich von der kationischen Polyaminosäure (b) unterscheidet.

10. Zusammensetzung nach einem der Ansprüche 1 bis 9, ferner umfassend (e) mindestens ein nichtionisches Tensid.

11. Zusammensetzung nach einem der Ansprüche 1 bis 10, wobei die Zusammensetzung eine kosmetische Zusammensetzung ist, vorzugsweise eine kosmetische Zusammensetzung vom Typ "Abspülen" und bevorzugter eine kosmetische Zusammensetzung vom Typ "Abspülen" für Haare.

12. Kosmetisches Verfahren zum Pflegen oder Konditionieren von Keratinfasern, vorzugsweise Haaren, umfassend den folgenden Schritt:
Auftragen, auf die Keratinfasern, der Zusammensetzung nach einem der Ansprüche 1 bis 11.

## Revendications

1. Composition pour le traitement de fibres kératiniques, de préférence des cheveux, comprenant :
(a) au moins un acide α-hydroxylé ou un sel de celui-ci ;
(b) au moins un acide polyaminé cationique comprenant au moins un groupe d'ammonium quaternaire et dérivé de protéines végétales ; et
(c) de l'eau,
dans laquelle
la quantité d'acide α-hydroxylé (a) ou du sel de celui-ci dans la composition est de 1 % en poids ou plus par rapport au poids total de la composition,
la quantité du ou des acides polyaminés cationiques (b) est de 0,2 % en poids ou plus, préférentiellement de 0,3 % en poids ou plus, plus préférentiellement de 0,5 % en poids ou plus, et encore plus préférentiellement de 0,8 % en poids ou plus, par rapport au poids total de la composition, et la quantité du ou des silicones dans la composition est de 1 % en poids ou moins, par rapport au poids total de la composition.

2. Composition selon la revendication 1, dans laquelle l'acide α-hydroxylé (a) possède deux groupes carboxyliques ou plus.

3. Composition selon la revendication 1 ou 2, dans laquelle l'acide α-hydroxylé (a) est choisi dans le groupe constitué par l'acide glycolique, l'acide lactique, l'acide malique, l'acide citrique, l'acide tartrique, l'acide mandélique, l'acide gluconique, l'acide mucique et un mélange de ceux-ci, et plus préférentiellement dans le groupe constitué par l'acide citrique, l'acide tartrique et un mélange de ceux-ci.

4. Composition selon l'une quelconque des revendications 1 à 3, dans laquelle la quantité du ou des acides α-hydroxylés (a) ou d'un sel de ceux-ci dans la composition est de 1 % à 10 % en poids, par rapport au poids total de la composition.

5. Composition selon l'une quelconque des revendications 1 à 4, dans laquelle l'acide polyaminé cationique (b) est choisi dans le groupe constitué par la protéine de blé hydrolysée au trimonium hydroxypropylé, la protéine de blé hydrolysée au stéardimonium hydroxypropylé, la protéine de blé hydrolysée au cocodimonium hydroxypropylé, la protéine de conchioline hydrolysée au trimonium hydroxypropylé, la protéine de soja hydrolysée au stéardimonium hydroxypropylé, la protéine de blé hydrolysée au trimonium hydroxypropylé, la protéine de soja hydrolysée au cocodimonium hydroxypropylé, et un mélange de celles-ci.

6. Composition selon l'une quelconque des revendications 1 à 5, dans laquelle la quantité du ou des acides polyaminés cationiques (b) cationiques dans la composition est de 20 % en poids ou moins, préférentiellement de 15 % en poids ou moins, plus préférentiellement de 10 % en poids ou moins, et encore plus préférentiellement de 5 % en poids ou moins, par rapport au poids total de la composition.

7. Composition selon l'une quelconque des revendications 1 à 6, dans laquelle la quantité de l'eau (c) dans la composition est de 50 % à 80 % en poids, préférentiellement de 55 % à 75 % en poids, et plus préférentiellement de 60 % à 70 % en poids, par rapport au poids total de la composition.

8. Composition selon l'une quelconque des revendications 1 à 7, dans laquelle le pH de la composition est inférieur à 7, préférentiellement inférieur à 6, et plus préférentiellement inférieur à 5.

9. Composition selon l'une quelconque des revendications 1 à 8, comprenant en outre (d) au moins un tensioactif cationique différent de l'acide polyaminé cationique (b).

10. Composition selon l'une quelconque des revendications 1 à 9, comprenant en outre (f) au moins un tensioactif non ionique.

11. Composition selon l'une quelconque des revendications 1 à 10, dans laquelle la composition est une composition cosmétique, préférentiellement une composition cosmétique de type rinçage, et plus préférentiellement une composition cosmétique capillaire de type rinçage.

12. Procédé cosmétique pour le soin ou le conditionnement des fibres kératiniques, de préférence des cheveux, comprenant l'étape de :
application sur les fibres kératiniques de la composition selon l'une quelconque des revendications 1 à 11.
